# EUROPÄISCHE PATENTANMELDUNG

(11) **EP 1 900 356 A1**
(43) Veröffentlichungstag der Anmeldung: **19.03.2008**
(21) Anmeldenummer: 06018855.4
(22) Anmeldetag: 08.09.2006
(51) Int. Cl.: A61K 8/97, A61Q 7/00, A61Q 5/00

(54) **Bockshornkleesamenextrakt**

(71) Anmelder: Arcon International GmbH, 78224 Singen (DE)
(72) Erfinder: Mai, Heinz, 78224 Singen (DE); Mai, Jutta, 78224 Singen (DE)
(74) Vertreter: Vossius & Partner

(57) **Zusammenfassung**

Die vorliegende Erfindung betrifft eine Zubereitung umfassend einen aus den Samen des Bockshornklees gewonnenen Extrakt, dadurch gekennzeichnet, dass der Extrakt Trigonellin und ein Gemisch aus Saponinen und/oder Sapogeninen enthält, wobei der Gesamtgehalt des Gemisches aus Saponinen und/oder Sapogeninen mindestens 8 Gew.-% beträgt.
Die erfindungsgemäße Zubereitung wird zum Anregen und/oder Wiederbeleben des Wachstums von Oberhautgebilden, insbesondere der Haare und Nägel sowie zur Förderung der Zellneubildung der Haut bereitgestellt.
Ferner wird die erfindungsgemäße Zubereitung zur Erhöhung der Haardichte, zur Stimulierung des Haarwuchses, zur Verhinderung des Haarverlusts oder zur Verhinderung der Bildung von Kopfschuppen bereitgestellt.
Die erfindungsgemäße Zubereitung ist auch zur Herstellung eines Arzneimittels zur Behandlung von krankhaft bedingtem Haarausfall, Alopecia areata, Alopecia diffusa, androgenetischer Alopezie, androgenetischem Haarausfall bei Frauen durch Behandlung hormonabhängiger Tumore, Alopecia praematura, Alopecia simplex oder Alopecia totalis, geeignet.

## Beschreibung

Die vorliegende Erfindung betrifft eine Zubereitung umfassend einen aus den Samen des Böckshornklees gewonnenen Extrakt, dadurch gekennzeichnet, dass der Extrakt Trigonellin und ein Gemisch aus Saponinen und/oder Sapogeninen enthält, wobei der Gesamtgehalt des Gemisches aus Saponinen und/oder Sapogeninen mindestens 8 Gew.-% beträgt.

Kräftige, gesunde Haare prägten das äußere Erscheinungsbild entscheidend. Volle kräftige Haare stehen für Gesundheit, Vitalität und Jugend. Es hat sich herausgestellt, dass gerade die Menschen in den Industrienationen besonders von Haarausfall geplagt sind. Waren in früheren Jahrzehnten vom Haarausfall mehr die Männer betroffen, so ist zu beobachten, dass dies auch zunehmend ein Problem für die Frauen darstellt.

Dem Menschen fallen durchschnittlich zwischen 60 bis 120 Haare pro Tag aus. Die Grenzen vom nicht-haarvermindernden Ausfall bis zum stark haarvermindernden Ausfall sind nicht starr gesetzt. Es hängt hier davon ab, wie leicht, in welchen Bereichen, wie viele Haare ausfallen. Als Faustregel gilt ein Ausfall von bis zu 100 Haaren pro Tag als normal, wenn der Haarausfall sich gleichmäßig über den ganzen Kopf verteilt. Konzentriert es sich jedoch auf einzelne Haarbereiche (Alopecia Areata) oder nur auf den Oberkopf (androgenetischer Haarausfall), gilt ein Ausfall von bis zu 100 Haaren pro Tag eher als besorgniserregend.

Androgenetischer Haarausfall (androgenetische Alopezie, alopecia androgenetica) ist ein erblich bedingter Haarausfall, für welchen das Hormon Dihydrotestosteron (DHT) verantwortlich gemacht wird. Wenn in der Kopfhaut viel DHT vorhanden ist und eine ererbte Übererüpfindlichkeit dafür besteht, wird die Wachstumsphase (Anagenphase) des Haares verkürzt. Aufgrund der Überempfindlichkeit gegenüber DHT verkümmern die Haarfolikel mit der Zeit. Bei männlichen Jugendlichen, bei denen eine familiäre Disposition vorherrscht, beginnt der genetisch bedingte Haarausfall meistens am Vorderkopf und an den Schläfen.
Diese Form des Haarausfalls wird Alopecia praematura oder simplex bezeichnet.

Durch die Behandlung hormonabhängiger Tumore wie Brustkrebs mit Aromatasehemmer können auch Frauen mit entsprechender genetischer Disposition androgenetischen Haarausfall entwickeln.

Unter Alopecia areata (Alopecia circumscripta; Pelade; Areata celsis; kreisrunder Haarausfall) versteht man einen runden, lokal begrenzten krankhaften Haarausfall. Die Kahlstellen sind glatt, eingesunken, nicht schuppend, und die Haarfollikel bleiben erhalten. Häufig bestehen zusätzlich Veränderungen der Fingernägel mit Grübchen, Rillen oder sandpapierartigen Aufrauhungen. Die genauen Ursachen dieser Krankheit ist noch nicht bekannt. Man geht jedoch von einer Störung des Immunsystems aus. Der Haarausfall kann schließlich weiter fortschreiten und zum Verlust aller Kopfhaare (Alopecia totalis) oder auch zum Verlust aller Körperhaare (Alopecia universalis) führen.

Beim diffusen Haarausfall (diffuse Alopezie; Alopecia diffusa) fallen die Haare vom gesamten Kopf ab. Dieser Haarausfall tritt häufiger bei Frauen als bei Männern auf. Ursachen können Hormonschwankungen, Schilddrüsenerkrankungen, Eisenmangel, Stress oder Infektionen sein. Einige Medikamente können ebenfalls zu Haarausfall führen. Auch Infektionen, wie z.B. Impetigo contaginosa, Karbunkel, Wundrose oder Gürtelrose, können zu einem zeitlich begrenzten Haarverlust führen.

Die genannten Formen des Haarausfalls werden versucht medikamentös zu behandeln. So wird z.B. der kreisrunde Haarausfall mit Diphenylcyclopropenon (DCP) therapiert. Diese Substanz ist jedoch relativ riskant und damit mit erheblichen Nebenwirkungen verbunden. Eine weitere Behandlungsmöglichkeit ist die sogenannte PUVA-Therapie mit ultravioletten Strahlen bestimmter Wellenlänge in Kombination mit Psoralen. Auch an spezifischen Immunsuppressiva besteht ein wissenschaftliches Interesse zur Behandlung des Haarausfalls. Ein weiterer Ansatzpunkt wären zum Beispiel spezielle Östrogenrezeptorantagonisten.

Ferner werden Antiandrogene wie Finasterid (Propecia^{®}) oder Dutasterid (Avodart^{®}) eingesetzt. Das Antihypertonikum Minoxidil zeigte als "Nebenwirkung" eine Hemmung des Haarausfalls. Schließlich werden auch Östrogene wie 17-alpha-Estradiol zur Hemmung des Haarausfalls eingesetzt oder cortisonhaltige Medikamente.

Die beschriebenen Therapieansätze weisen zwar eine gewisse Wirksamkeit gegen den Haarausfall auf, bergen jedoch auch ein erhebliches Nebenwirkungspotenzial in sich. So ist schließlich auch zu bedenken, dass diese Substanzen meist ein Leben lang einzunehmen bzw. aufzutragen sind, da bei Absetzung der Substanzen das neugewonnen Haar wieder ausfällt.

Zusammenfassend lässt sich sagen, dass die gegenwärtig gebräuchlichen Methoden zur Behandlung des Haarausfalls noch nicht optimal sind.

Aufgabe der Erfindung ist es somit eine Zubereitung mit hoher Wirksamkeit und einem geringen Nebenwirkungsprofil bereitzustellen, die eine Einnahme über einen langen Zeitraum erlaubt.

Erfindungsgemäß wird zur Lösung dieser Aufgabe eine Zubereitung umfassend einen aus den Samen des Bockshornklees gewonnenen Extrakt, dadurch gekennzeichnet, dass der Extrakt Trigonellin und ein Gemisch aus Saponinen und/oder Sapogeninen enthält, wobei der Gesamtgehalt des Gemisches aus Saponinen und/oder Sapogeninen mindestens 8 Gew.-% beträgt, bereitgestellt.

So hat sich gegenüber dem Stand der Technik herausgestellt, dass der erfindungsgemäße Extrakt enthaltend Trigonellin und ein Gemisch aus Saponinen und/oder Sapogeninen von mindestens 8 Gew.-% gewonnen aus dem Samen des Bockshornklees eine verbesserte Wirkung auf das Wachstums von Oberhautgebilden, wie der Haare und Nägel, sowie auf die Zellneubildung der Haut hat.

DE 31 18 882 betrifft einen Knoblauchextrakt als Mittel gegen Haarausfall und zur Förderung des Haarwuchses, der mit einem Aminosäuregemisch kombiniert werden kann. EP 0 469 007 B1 und DE 4 012 148 A1 beschreiben ein Mittel zur Nagel-, Haut- und Haarpflege, enthaltend Trigonellin und Vitamin B6.
DE 36 03 601 C2 betrifft die Verwendung eines Bockshornkleesamenextraktes zur Wiederbelebung und zum Anregen und Verstärken des Haarwuchses bei Lebewesen.
EP 0 289 639 B2 betrifft die Verwendung von Trigonellin zur Wiederbelebung des Haarwuchses.
EP 0 859 582 B1 betrifft ein Mittel zur Pflege der Haut und der Haare enthaltend Trigonellin bzw. Trigonellinsäure und einen Extrakt von Ginseng.

DE 39 15 535 A1 betrifft ein Mittel zur Bekämpfung von Hauterkrankungen enthaltend Trigonellin bzw. Trigonellinsäure.

Während sich die im Stand der Technik aufgefundenen Dokumente auf das im Bockshornkleesamen enthaltene Trigonellin bzw. Trigonellinsäure fokussieren, betrifft keines der Dokumente die erfindungsgemäße Kombination aus Trigonellin und einem Gemisch aus Saponinen und/oder Sapogeninen von mindestens 8 Gew.-%, die aus den Samen des Bockshornklees gewonnen wird.

Bockshornklee *(Trigonella foenum-graecum)* gehört zur Familie der Hülsenfrüchtler (Fabaceae), Unterfamilie Schmetterlingsblütler (Faboideae). Es ist eine einjährige, bis zu 60 cm hohe Pflanze.

Das Alkaloid Trigonellin (Coffearin, N-Methylbetain der Nicotinsäure) führt zu einer Vasodilation der peripheren Blutgefässe, so dass dadurch die Versorgung der Haarfollikel mit für das Haarwachstum notwendigen Nährstoffen wieder eingeleitet wird. Gemäß der vorliegenden Erfindung kann Trigonellin als Monohydrat oder in Form eines pharmazeutisch verträglichen Salzes, insbesondere in Form des Hydrochlorid-Salzes vorliegen.

Saponine sind Glycoside, die durch saure oder enzymatische Hydrolyse in Sapogenine (Aglykone) und Zucker gespalten werden. Die im Bockshornkleesamen enthaltenen Saponine und/oder Sapogenine sind 3,26-Bisglykoside mit Δ⁴=Furosten-, Δ⁵-Furosten- oder 5α-Furostan-Grundkörper; Trigofoenosid A bis G, Spirostanolglycoside, Diosgenin, Diocin, Yamogenin, Neogitogenin, Gitogenin, Trigoneoside Ia, IIa, IIb, IIIa, IIIb, IVa, Va, Vb, VI, VIIb, VIIIb, IX; Glykosid D; Trigonellosid C; und Trigoneoside Ia, Ib, Va, Xa, Xb, XIb, XIIa, XIIb, XIIIa, und deren Derivate. Saponine und/oder Sapogenine sind bekannt für ihre hämolytische, expektorische und diuretische Wirkung.

Derivate der Saponine und/oder Sapogenine sind auf dem Fachgebiet allgemein bekannt. Derivate der Saponine und/oder Sapogenine können unter anderem Ester, Amide, Peptide, Ether und Prodrugs der Saponine und/oder Sapogenine umfassen.

Unter Prodrugs sind Derivate der Saponine und/oder Sapogenine zu verstehen, die erst im Körper zu dem eigentlich pharmakologisch aktiven Wirkstoff verstoffwechselt werden, wie z.B. Ester- oder Amidderivate der Saponine und/oder Sapogenine.

Der erfindungsgemäßen Extrakt aus dem Samen des Bockshornklees ist dadurch gekennzeichnet, dass dieser Trigonellin und ein Gemisch aus Saponinen und/oder Sapogeninen enthält, wobei der Gesamtgehalt des Gemisches aus Saponinen und/oder Sapogeninen mindestens 8 Gew.-% beträgt.

In dem erfindungsgemäßen Extrakt aus dem Samen des Bockshornklees können gegebenenfalls zusätzlich eine oder mehrere Substanzen, die aus ätherischen Öle, Flavonoiden, Isoflavonoiden, Schleimstoffen, Lipiden, Sterolen, Phytosterinen, 25α-Spirostan, Enzymen, Peptiden, Aminosäuren, Fettsäuren, ungesättigte Fettsäuren, insbesondere Ω-3-Fettsäuren und Ω-6-Fettsäuren, Proteinen und Proteaseinhibitoren ausgewählt sind, enthalten sein.

Insbesondere können enthalten sein: Ätherische Öle wie 4,5-Dimethyl-3-hydroxy-2-(5H)-furanon (Sotolon), 3-Hydroxy-4-methyl-2(5H)-furanon, Dihydroactinidindiolid, Dihdyrobenzofuran, n-Hexanol; Diosgenin-Peptidester Foenugraecin; Flavoniode wie Glykosylflavone wie z.B. Vitexin, Isovitexin, Vicenin-1, Vicenin-2, Orientin- und Isoorientinarabinosid und Saponaretin; Isoflavonoide, wie Rotenoide; Schleimstoffe, wie z.B. β-(1→4)-Mannane, substituiert mit α-(1→6)-Galaktosylresten; Lipiden, wie z.B. Triacylglycerole, Phospholipide, Glycolipide; Sterole, wie z.B. β-Sitosterol und Cholserin; Proteine, wie z.B. L-Lysin und L-Tryptophan; Proteinaseinhibitoren , wie z.B. Bowman-Birk-Proteinaseinhibitoren sowie Polypeptide, die Trypsin und Chymotrypsin hemmen.

Die erfindungsgemäße Zusammensetzung wird aus den Samen des Bockshornklees durch Perkolation gewonnen. Bevorzugt handelt es sich bei diesem Extraktionsverfahren um ein Batterieumlaufverfahren, bei welchem keine Auswaschphase und nur eine Extraktionsphase erfolgt. Als Extraktionsmittel dient Ethanol unterschiedlicher Konzentration und Wasser (Ethanol/Wasser-Gemisch). Die Extraktion des Bockshornklee-Samens zur Bereitstellung der erfindungsgemäßen Zusammensetzung erfolgt nach dem Zweistufen-Prinzip. Hierfür werden die Extraktionsmittel unterschiedlichen Ethanolgehalts chronologisch nacheinander eingesetzt, so dass ein Vollextrakt mit lipophilen und hydrophilen Fraktionen des Samens resultiert. Bevorzugt wird der Samen des Bockshornklees mit einem Ethanol/Wasser-Gemisch von 90 Vol.-% Ethanol und anschließend mit einem Ethanol/Wasser-Gemisch von 50 Vol.-% Ethanol extrahiert. Die Perkolation erfolgt über mehrere Stunden bei einer Temperatur von 40 bis 60 °C.

Durch das beschriebene Verfahren kann ein Vollextrakt aus den Samen des Bockshornklees, enthaltend Trigonellin und ein Gemisch aus Saponinen und/oder Sapogeninen von mindestens 8 Gew.-%, mit lipohilen und hydrophilen Anteilen zur Bereitstellung der erfindungsgemäßen Zusammensetzung gewonnen werden.

Der Extrakt zur Bereitstellung der erfindungsgemäßen Zusammensetzung liegt bevorzugt als Trockenextrakt vor. Der Trockenextrakt kann durch Verdampfen der Extraktionsflüssigkeit, z.B. in einem Destilliergefäß auf einem Wasserbad, gewonnen werden.

Der Gehalt des Gemisches aus Saponinen und/oder Sapogeninen wurde als Gesamtsapogenine mit Diosgenin als Referenzsubstanz nach Ph. Eur. (Pharmacopoeia Europaea) 5.4, Monographie: Butcher's Broom bestimmt.

Die angegebenen Prozentangaben [Gew.-%] beziehen sich entsprechend auf den Anteil an den im erfindungsgemäßen Extrakt enthaltenen Sapogeninen und/oder Saponinen bezogen auf die Gesamtextrakteinwaage.

Die verbesserte Wirkung ist auf die im Extrakt enthaltene Kombination aus Trigonellin und dem Gemisch aus Saponinen und/oder Sapogeninen von einem Mindestgehalt von 8 Gew.-% zurückzuführen.

Der genaue Mechanismus der diesem erfindungsgemäßen Extrakt zugrundegelegten Wirkung ist bisher noch unbekannt.

Es ist jedoch davon auszugehen, dass diese verbesserte Wirkung durch die optimale Synergie der Kombination aus den Inhaltsstoffen Trigonellin und dem Gemisch aus Saponinen und/oder Sapogeninen von mindestens 8 Gew.-% zustande kommt.

Dieser verbesserte, synergistische Effekt kann wie folgt erklärt werden:

Die Inhaltsstoffe des Bockshornkleesamens enthalten unter anderem Trigonellin, Diosgenin-Peptidester sowie in nennenswerten Mengen Saponine und Sapogenine.
Diese Stoffe besitzen entweder eine physiologische estrogene oder eine antiandrogene Wirkung.

Bei rund 90% aller Männer, die unter Haarverlust klagen, liegt eine androgenetische Alopezie vor. Sie betrifft rund 40% aller 40-jährigen und die Hälfte aller 50-jährigen Männer. Im Alter von 80 Jahren haben mehr als 80% der Männer eine Alopezie. Ursache hierfür ist, dass die Kopfhaut eine erhöhte Aktivität der 5-Alpha -Reductase und damit eine erhöhte Konzentration von Dihydrotestosteron (DHT) besitzt. Für ein normales Haarwachstum sind jedoch normale Testosteronspiegel und niedrige DHT-Spiegel erforderlich. Es ist daher zu vermuten, dass die Inhaltsstoffe des Bockshornkleesamens, speziell die Kombination aus Trigonellin und einem Gemisch aus Saponinen und/oder Sapogeninen, insbesondere der Diosgenin-Derivate, eine gewisse antiandrogene bzw. estrogene Wirkung besitzen.

Estrogene sind die natürlichen Gegenspieler des Testosterons bzw. von DHT. Entweder wird dabei weniger Testosteron gebildet und damit auch weniger DHT oder die genannten Kombination der Inhaltsstoffe des Bockshornsamens haben einen direkten Einfluß auf die Aktivität der 5-Alpha-Reductase.

Aber auch Frauen sind von der aridrogenetischen Alopezie betroffen. Neben einer erblichen Komponente spielt dabei insbesondere in und nach den Wechseljahren ein Absinken der Estrogenspiegel eine wichtige Rolle. Dies kann dazu führen, dass sich vermehrt männliche Sexualhormone durchsetzen. Durch antiandrogene bzw. estrogene Nährstoffe (z.B. auch Phytoestrogene) können diese Vorgänge wieder normalisiert werden. Es ist deshalb zu vermuten, dass die erfindungsgemäße Kombination aus den Inhaltsstoffen des Bockshornkleesamens die eine oder die andere physiolgische Wirkung besitzen.

Die nachfolgend beschriebenen Inhaltsstoffe zeigen eine zusätzliche positive Wirkung auf die erfindungsgemäße Zusammensetzung (Extrakt).

Zink spielt eine wichtige Rolle im Stoffwechsel des Cysteins, der wichtigsten Aminosäuren für den Aufbau des Haarkeratins. Bei Zinkmangel ist das Haar häufig dünn, farblos und brüchig. Neben den Haaren können sich aber auch Veränderung an den Nägeln in Form weißer Flecken bemerkbar machen.

Calciumpantothenat ist ein essenzielles B-Vitamin mit einer wichtigen Rolle im Stoffwechsel des teilungsaktiven Gewebes und für dessen Regenerationsprozess.

Vitamin B6 fördert die Umwandlung von Tryptophan in Picolinsäure. Picolinsäure ist ein wichtiger Transporter für Zink. Damit hängt die Zinkverwertung des Organismus unter anderem auch von dem Vitamin B6 ab. Zink verbessert aber auch die Struktur des Haares.

Vitamin E verbessert die Durchblutung der Kopfhaut und hilft damit gegen Haarausfall, insbesondere nach Bestrahlung (z. B. UV Belastung). Als antioxidative Vitamine schützen die Vitamine E und C das Haarfollikel vor oxidativen Belastungen.

Kupfer zeigt einen synergistischen Effekt mit Zink.

Biotin wird bei Haarausfall und Nagelbrüchigkeit therapeutisch eingesetzt. Biotin und Zink aktivieren den Stoffwechsel der Haarfollikel und verbessern die Qualität des Keratins.

Nicotinamid verbessert die Durchblutung der Kopfhaut.

Selen schützt gemeinsam mit den antioxidativen Vitaminen E und C das Haarfollikel vor oxidativen Belastungen wie z.B. zu viel Sonnenlicht, ständiges Waschen und Chemikalien, die das Haar und die Kopfhaut schwächen und austrocknen können.

Jod ist ein Mineralstoff, der für einen normalen Zellstoffwechsel benötigt wird. Jod wird von der Schilddrüse für die Synthese und Absonderung von Hormonen benötigt. Ein Jodmangel tritt häufig im Zusammenhang mit Fettstoffwechselstörungen auf Ferner hat sich gezeigt, dass ein Jodmangel zu trockener Haut führen kann, was wiederum in direktem Zusammenhang mit dem Haarwachstum steht.

Die erfindungsgemäße Zubereitung, umfassend einen aus den Samen des Bockshornklees gewonnenen Extrakt, dadurch gekennzeichnet, dass der Extrakt Trigonellin und ein Gemisch aus Saponinen und/oder Sapogeninen enthält, wobei der Gesamtgehalt des Gemisches aus Saponinen und/oder Sapogeninen mindestens 8 Gew.-% beträgt, kann als Arzneimittel, Nahrungsmittel, Nahrungsergänzungsmittel oder Diätetikum vorliegen.

Die erfindungsgemäße Zubereitung wird zum Anregen und/oder Wiederbeleben des Wachstums von Oberhautgebilden, insbesondere der Haare und Nägel sowie zur Förderung der Zellneubildung der Haut bereitgestellt.

Ferner wird die erfindungsgemäße Zubereitung zur Erhöhung der Haardichte, zur Stimulierung des Haarwuchses, zur Verhinderung des Haarverlusts oder zur Verhinderung der Bildung von Kopfschuppen bereitgestellt.

Die erfindungsgemäße Zubereitung ist auch zur Herstellung eines Arzneimittels zur Behandlung von krankhaft bedingtem Haarausfall, Alopecia areata, Alopecia diffusa, androgenetischer Alopezie, androgenetischem Haarausfall bei Frauen durch Behandlung hormonabhängiger Tumore, Alopecia praematura, Alopecia simplex oder Alopecia totalis, geeignet.

Eine geeignete Dosierung hängt von verschiedenen Faktoren, wie z.B. Köpergröße, Gewicht, Körperoberfläche, Alter, Geschlecht, Tageszeit, Art der Verabreichung, allgemeinen Gesundheitszustand des Patienten und anderen eingenommenen Medikamenten ab.

Es hat sich gezeigt, dass die verbesserte Wirkung der erfindungsgemäßen Zubereitung gewonnen aus den Samen des Bockshornklees, umfassend Trigonellin und ein Gemisch aus Saponinen und/oder Sapogeninen von mindestens 8 Gew.-%, insbesondere bei einer Dosierung von 100 mg bis 2000 mg Bockshornkleesamen-Extrakt auftritt.

In einer anderen Ausführungsform wird die erfindungsgemäße Zubereitung gewonnen aus den Samen des Bockshornklees, umfassend Trigonellin und ein Gemisch aus Saponinen und/oder Sapogeninen von mindestens 8 Gew.-%, in einer Dosierung von 100 mg bis 1000 mg Bockshornkleesamen-Extrakt verabreicht.

In einer bevorzugten Ausführungsform wird die erfindungsgemäße Zubereitung gewonnen aus den Samen des Bockshornklees, umfassend Trigonellin und ein Gemisch aus Saponinen und/oder Sapogeninen von mindestens 8 Gew.-%, in einer Dosierung von 100 mg bis 500 mg Bockshornkleesamen-Extrakt verabreicht.

In einer stärker bevorzugten Ausführungsform wird die erfindungsgemäße Zubereitung gewonnen aus den Samen des Bockshornklees, umfassend Trigonellin und ein Gemisch aus Saponinen und/oder Sapogeninen von mindestens 8 Gew.-%, in einer Dosierung von 100 mg bis 200 mg Bockshornkleesamen-Extrakt verabreicht.

Die erfindungsgemäße Zubereitung, umfassend einen aus den Samen des Bockshornklees gewonnenen Extrakt, dadurch gekennzeichnet, dass der Extrakt Trigonellin und ein Gemisch aus Saponinen und/oder Sapogeninen enthält, wobei der Gesamtgehalt des Gemisches aus Saponinen und/oder Sapogeninen mindestens 8 Gew.-% beträgt, kann gegebenenfalls zusätzlich eine oder mehrere Substanzen, die aus Vitaminen, Mineralstoffen, Aminosäuren, Spurenelementen, Antioxidantien, Coenzymen, Fettsäuren, Co-faktoren, Lignanen, Phytoöstrogenen und Mikronährstoffen ausgewählt sind, umfassen.

Die erfindungsgemäße Zubereitung, kann gegebenenfalls zusätzlich eine oder mehrere Substanzen ausgewählt aus Vitamin A, B, C, D, E, H, K, Nicotinamid, Panthotensäure und Folsäure; Natrium, Kalium, Magnesium, Calcium, Phosphor, Chlorid; Glycin, L-Alanin, L-Serin, L-Threonin, L-Valin, L-Leucin, L-Isoleucin, L-Asparaginsäure, L-Asparagin, L-Glutaminsäure, L-Glutamin, L-Arginin, L-Lysin, L-Hydroxylysin, L-Ornithin, L-Citrullin, L-Cystein, L-Cystin, L-Methionin, L-Tyrosin, L-Phenylalanin, L-Tryptophan, L-Histidin, L-Prolin und L-Hydroxyprolin; Eisen, Jod, Fluorid, Zink, Kupfer, Mangan, Selen, Chrom und Molybdän; β-Carotin, Lycopin, Lutein und Zeaxanthin; Coenzym Q10, Adenosintriphosphat, Cytidintriphosphat, Guanosintriphosphat, Uridintriphosphat, Biotin, Coenzym A, Methylcobalamin, Liponsäure, Phyllohydrochinon, Pyridoxalphosphat, Retinylphosphat, Tetrahydrofolsäure, Thiaminpyrophosphat, Flavin-Adenin-Dinucleotid (FAD), Flavin-Mono-Nucleotid (FMN), Nicotinamid-Adenin-Dinucleotid (NAD) und Nicotinamid-Adenin-Dinucleotidphosphat (NADP); Ölsäure, Linolsäure, Linolensäure, Ω-6-Fettsäuren und Ω -3-Fettsäuren, Cholinhydrogentartrat, Colaminphosphat, Quercetin, Siliciumdioxid, Taurin sowie deren Salze, umfassen.

Die erfindungsgemäße Zubereitung, gewonnen aus den Samen des Bockshornklees, umfassend Trigonellin und ein Gemisch aus Saponinen und/oder Sapogeninen von mindestens 8 Gew.-%, kann auf die für den Fachmann bekannte Weise in geeigneten Darreichungsformen bzw. Dosierungseinheiten, die gegebenenfalls die auf dem Fachgebiet bekannten pharmazeutisch verträglichen Hilfsstoffe bzw. Excipienten umfassen, verabreicht werden, wie zum Beispiel oral, cutan, transdermal, transmukosal, lokal oder topisch gegebenenfalls über Iontopherese oder sublingual.

Die erfindungsgemäße Zubereitung kann nach für den Fachmann bekannten Techniken unter Verwendung von üblicherweise verwendeten Hilfsstoffen formuliert werden.

Die Darreichungsformen der erfindungsgemäßen Zubereitung können zum Beispiel Formulierungshilfsstoffe wie Füllmittel (Träger), Sprengmittel, Bindemittel, Fließregulierungsmittel, Gleitmittel, Emulgatoren, Solubilisatoren, Benetzer, Entschäumer, Viskositäts- und Konsistenzbeeinflusser, Gelbildner, Lösungsmittel, Lösungsvermittler, Sorptionsmittel, Weichmacher, Trennmittel, Feuchthaltemittel, Adsorptionsmittel, Resorptionsbeschleuniger, Filmbildner, Süßungsmittel wie zum Beispiel Zucker, Zuckeraustauschstoffe und künstliche Süßstoffe, Säuerungsmittel, Aromastoffe, Farbstoffe, Antioxidantien, Synergisten, Konservierungsmittel, Geschmackskorrigentien und Färbemittel umfassen. Weitere gebräuchliche Hilfsstoffe werden ferner in "Remington's Pharmaceutical Science, 15. Auflage, Mack Publishing Co., New Jersey (1991) beschrieben.

Die erfindungsgemäße Zubereitung kann in verschiedenen Produkten enthalten sein. So können z.B. Nahrungsmittel, diätetisches Lebensmittel, (ergänzende) bilanzierte Diät, Nahrungsergänzungsmittel, Medizinprodukt, feste, flüssige oder halbfeste Zubereitung mit der erfindungsgemäßen Zubereitung aus Trigonellin und einem Gemisch aus Saponinen und/oder Sapogeninen von mindestens 8 Gew.-%, ergänzt werden.

Ferner kann die erfindungsgemäße Zubereitung als Flüssigextrakt, Dickextrakt, Trockenextrakt, Suspension, Emulsion, Pulver oder Granulat vorliegen.

Die erfindungsgemäße Zubereitung kann auch in Form eines Getränkes, einer Ampulle oder Trinkampulle bereitgestellt werden.

Die erfindungsgemäße Zubereitung kann auch in Form einer Tablette, Kapsel, eines Dragee, Gummibonbons, Bonbons, Schaumzuckerware, in Form von Müsli oder Kaugummi bereitgestellt werden.

Ferner kann die erfindungsgemäße Zubereitung auch topisch verabreicht werden, z.B. als Salbe, Creme, Gel, Lotion, Mixtur, Tinktur, Shampoo, Haarspray, Haarspülung oder Pflaster.

Bevorzugte Ausführungsformen sind nachfolgend angegeben:

Die erfindungsgemäße Zubereitung umfasst einen aus den Samen des Bockshornklees gewonnenen Extrakt, welcher dadurch gekennzeichnet ist, dass Trigonellin und ein Gemisch aus Saponinen und/oder Sapogeninen enthalten sind, wobei der Gesamtgehalt des Gemisches aus Saponinen und/oder Sapogeninen vorzugsweise mindestens 10 Gew.-%, stärker bevorzugt mindestens 11 Gew.-% beträgt. Vorzugsweise sind in der erfindungsgemäßen Zubereitung das Saponin und/oder Sapogenin Diosgenin, Diocin oder ein Diosgenin-Peptidester enthalten.

Der Extrakt, enthaltend Trigonellin und ein Gemisch aus Saponinen und/oder Sapogeninen von mindestens 8 Gew.-%, zur Bereitstellung der erfindungsgemäßen Zubereitung liegt bevorzugt in Form eines Trockenextraktes vor.

Bevorzugte Formulierungen der erfindungsgemäßen Zubereitung enthalten pro Darreichungsform, wie z.B. Tablette, Kapsel oder Dragee

| | |
|---|---|
| Bockshornkleesamen-Extrakt | 100 - 2000 mg |
| Ascorbinsäure | 30 - 200 mg |
| D-α-Tocoplierol | 1 - 30 mg |
| Nicotinamid | 1 - 20 mg |
| Calciumpantothenat | 1 - 20 mg |
| Zinkoxid | 1 -100 mg |
| Pyridoxin | 0,1-5 mg |
| Thiamin | 0,1-5 mg |
| Riboflavin | 0,1 - 5 mg |
| Kupfer | 0,01-2 mg |
| Biotin | 50 - 5000 µg |
| Folsäure | 50 - 300 µg |
| Jod | 10 - 200 µg |
| Selen | 5 *-* 100 µg |

Eine stärker bevorzugte Formulierung der erfindungsgemäßen Zubereitung enthält pro Darreichungsform

| | |
|---|---|
| Bockshornkleesamen-Extrakt | 150 mg |
| Ascorbinsäure | 60 mg |
| D-α-Tocopherol | 10 mg |
| Nicotinamid | 9 mg |
| Calciumpantothenat | 3 mg |
| Zinkoxid | 2,5 mg |
| Pyridoxin | 1 mg |
| Thiamin | 0,7 mg |
| Riboflavin | 0,8 mg |
| Kupfer | 0,25 mg |
| Biotin | 150 µg |
| Folsäure | 100 µg |
| Jod | 37,5 µg |
| Selen | 15 µg |

Die erfindungsgemäße Zubereitung wird bevorzugt oral in Form einer Kapsel verabreicht.

Die Tagesdosis der erfindungsgemäßen Zubereitung beträgt mindestens 200 mg, bevorzugt mindestens 300 mg Bockshornkleesamen-Extrakt. Eine Formulierung der erfindungsgemäßen Zubereitung, wird bevorzugt 2 mal täglich verabreicht. Insbesondere wird eine Formulierung der erfindungsgemäßen Zubereitung, enthaltend 150 mg Bockshornkleesamen-Extrakt, in einer Kapsel 2 mal täglich verabreicht.

### Studie

Die Effektivität der erfindungsgemäßen Zubereitung wurde in einer monozentrischen, randomisierten doppelblinden und placebokontrollierten Studie demonstriert. Ziel dieser Studie war es die Wirksamkeit der erfindungsgemäßen Zubereitung, gewonnen aus den Samen des Bockshornklees, umfassend Trigonellin und einem Gemisch aus Saponinen und/oder Sapogeninen von mindestens 8 Gew.-%, gegen Haarausfall zu untersuchen. Die Wirksamkeit wurde über verschiedene Zeitpunkte im Vergleich zu Placebo untersucht. Die folgenden Parameter wurde mittels Bildanalyse ausgewertet.
- Haardichte
- Haarstärke
- Haarwachstumsrate
- Anagen/Telogen-Rate

Zusätzliche wurde von einem Dermatologen und den Probanden die Wirksamkeit der erfindungsgemäßen Zubereitung hinsichtlich der Parameter Haardichte, Haarverlust und kräftiges Haar bewertet.

60 Probanden (30 männliche und 30 weibliche) mit schwachen bis mäßigen Haarausfall haben einmal täglich zwei Kapseln der erfindungsgemäßen Zubereitung über einen Zeitraum von sechs Monaten eingenommen. Es wurden nach zwei Monaten sechs Probanden zusätzlich eingeschlossen, die an der Studie von Visit 1 bis 8 teilgenommen haben. Jeweils 20 der Probanden pro Geschlecht haben das Verum und jeweils 10 das Plazebo eingenommen.

Bei Baseline (Visit 1 und 2) und den Zeitpunkten von 2 Monaten (Visit 4 und 5), 4 Monaten (Visit 7 und 8) und 6 Monaten (Visit 10 und 11), gab es eine dermatologischen Einschätzung des Haarverlustes, einen Teilnehmerfragebogen und eine Einschätzung der Haardichte (n/cm²), der Haarstärke (µm), der Haarwachstumsrate (mm/Tag) und der Anagen/Telogen-Rate.

Für die Einschätzung der Haardichte, Haarstärke, der Haarwachstumsrate und der Anagen/Telogen-Rate, die mittels eines Videomikroskops und anschließender Bildanalyse erfolgte, musste das Haar auf einen Bereich von ungefähr 2 cm² rasiert und gefärbt werden. An den Rasiertagen wurde die Haarlänge kontrolliert und mit Baseline verglichen. Die vier Parameter (Haardichte, Haarstärke, Haarwachstumsrate, Anagen/Telogen-Rate) wurden immer drei Tage nach dem Rasieren bestimmt. Dazu wurde die Färbung an diesem Tag erneuert.

Zusätzliche "Compliance-Visits" fanden nach einem Monat (Visit 3), drei Monaten (Visit 6) und fünf Monaten (Visit 9) statt. Jede mögliche ärztliche Medikation, die während der Studie genommen wurde und die nicht in den Ausschlusskriterien verzeichnet war, wurde mit Handelsnamen am Anfang der Studie (Visit 1) dokumentiert. Die Teilnehmer wurden nach Änderung in der begleitenden Medikation an Besuchen/Visit 3, 4, 6, 7, 9 und 11 befragt.

Ein Fragebogen über die Gewohnheiten der Teilnehmer (z. B. Raucher/Nichtraucher, Kaffeeverbraucher, Fleischverbraucher, Vegetarier/Nichtvegetarier) sowie Gewicht und Größe wurde von dem Probanden in Visit 1 ausgefüllt. Ein abschließender Fragebogen hinsichtlich der Erfahrung der Probanden mit dem Testprodukt in der Studie wurde von diesen am Ende der Studie ausgefüllt. Diese Fragebögen wurden zur Studienkoordination zur weiteren Analyse bearbeitet.

### Ergebnisse und Schlussfolgerungen

Für die erfindungsgemäße Zusammensetzung ergab sich bei allen Parametern, wie der Haardichte (Bildanalyse), Haarwachstumsrate, Verhältnis anagener/telogener Haare, sowie der Bewertung des Haarverlusts (Dermatologe und Proband), der Haardichte (Proband) und kräftiges Haar (Dermatologe und Proband) eine Verbesserung im Studienverlauf. Die erfindungsgemäße Zusammensetzung zeigte vor allem signifikante Unterschiede bei den Parametern Haardichte (Bildanalyse) und Haardichte (Proband).

## Patentansprüche

1. Zubereitung umfassend einen aus den Samen des Bockshornklees gewonnenen Extrakt, **dadurch gekennzeichnet, dass** der Extrakt Trigonellin und ein Gemisch aus Saponinen und/oder Sapogeninen enthält, wobei der Gesamtgehalt des Gemisches aus Saponinen und/oder Sapogeninen mindestens 8 Gew.-% beträgt.

2. Zubereitung nach Anspruch 1, wobei der Gesamtgehalt des Gemisches aus Saponinen und/oder Sapogeninen mindestens 10 Gew.-% beträgt.

3. Zubereitung nach Anspruch 1 oder 2, wobei die Saponine und/oder Sapogenine aus 3,26-Bisglykoside mit Δ⁴-Furosten-, Δ⁵-Furosten- oder 5α-Furostan-Grundkörper; Trigofoenosid A bis G, Spirostanolglycoside, Diosgenin, Diocin, Yamogenin, Neogitogenin, Gitogenin, Trigoneoside Ia, IIa, IIb, IIIa, IIIb, IVa, Va, Vb, VI, VIIb, VIIIb, IX; Glykosid D; Trigonellosid C; und Trigoneoside Ia, Ib, Va, Xa, Xb, XIb, XIIa, XIIb, XIIIa, und deren Derivate, ausgewählt sind.

4. Zubereitung nach Anspruch 3, wobei das Saponin und/oder Sapogenin Diosgenin, Diocin oder ein Diosgenin-Peptidester ist.

5. Zubereitung nach einem der vorstehenden Ansprüche, wobei der Extrakt gegebenenfalls zusätzlich eine oder mehrere Substanzen, die aus ätherischen Öle, Flavonoiden, Isoflavonoiden, Schleimstoffen, Lipiden, Sterolen, Phytosterinen, 25α-Spirostan, Enzymen, Peptiden, Aminosäuren, Fettsäuren, ungesättigte Fettsäuren, insbesondere Ω-3-Fettsäuren und Ω-6-Fettsäuren, Proteinen und Proteaseinhibitoren ausgewählt sind, umfasst.

6. Zubereitung nach einem der vorstehenden Ansprüche, die gegebenenfalls zusätzlich eine oder mehrere Substanzen, die aus Vitaminen, Mineralstoffen, Aminosäuren, Spurenelementen, Antioxidantien, Coenzymen, Fettsäuren, Co-faktoren, Lignanen, Phytoöstrogenen und Mikronährstoffen ausgewählt sind, umfasst.

7. Zubereitung nach Anspruch 5, wobei die Substanzen aus Vitamin A, B, C, D, E, H, K, Nicotinamid, Panthotensäure und Folsäure; Natrium, Kalium, Magnesium, Calcium, Phosphor, Chlorid; Glycin, L-Alanin, L-Serin, L-Threonin, L-Valin, L-Leucin, L-Isoleucin, L-Asparaginsäure, L-Asparagin, L-Glutaminsäure, L-Glutamin, L-Arginin, L-Lysin, L-Hydroxylysin, L-Ornithin, L-Citrullin, L-Cystein, L-Cystin, L-Methionin, L-Tyrosin, L-Phenylalanin, L-Tryptophan, L-Histidin, L-Prolin und L-Hydroxyprolin; Eisen, Jod, Fluorid, Zink, Kupfer, Mangan, Selen, Chrom und Molybdän; β-Carotin, Lycopin, Lutein und Zeaxanthin; Coenzym Q₁₀, Adenosintriphosphat, Cytidintriphosphat, Guanosintriphosphat, Uridintriphosphat, Biotin, Coenzym A, Methylcobalamin, Liponsäure, Phyllohydrochinon, Pyridoxalphosphat, Retinylphosphat, Tetrahydrofolsäure, Thiaminpyrophosphat, Flavin-Adenin-Dinucleotid (FAD), Flavin-Mono-Nucleotid (FMN), Nicotinamid-Adenin-Dinucleotid (NAD) und Nicotinamid-Adenin-Dinucleotidphosphat (NADP); Ölsäure, Linolsäure, Linolensäure, Ω-6-Fettsäuren und Ω -3-Fettsäuren, Cholinhydrogentartrat, Colaminphosphat, Quercetin, Siliciumdioxid, Taurin sowie deren Salze ausgewählt sind.

8. Zubereitung nach einem der vorstehenden Ansprüche, enthaltend pro Darreichungsform
| | |
|---|---|
| Bockshornkleesamen-Extrakt | 100 - 2000 mg |
| Ascorbinsäure | 30-200 mg |
| D-α-Tocopherol | 1 - 30 mg |
| Nicotinamid | 1 - 20 mg |
| Calciumpantothenat | 1 - 20 mg |
| Zinkoxid | 1 - 100 mg |
| Pyridoxin | 0,1-5 mg |
| Thiamin | 0,1 - 5 mg |
| Riboflavin | 0,1 - 5 mg |
| Kupfer | 0,01-2 mg |
| Biotin | 50 - 5000 µg |
| Folsäure | 50 - 300 µg |
| Jod | 10 - 200 µg |
| Selen | 5 - 100 µg |

9. Zubereitung nach einem der vorstehenden Ansprüche, enthaltend pro Darreichungsform
| | |
|---|---|
| Bockshornkleesamen-Extrakt | 150 mg |
| Ascorbinsäure | 60 mg |
| D-α-Tocopherol | 10 mg |
| Nicotinamid | 9 mg |
| Calciumpantothenat | 3 mg |
| Zinkoxid | 2,5 mg |
| Pyridoxin | 1 mg |
| Thiamin | 0,7 mg |
| Riboflavin | 0,8 mg |
| Kupfer | 0,25 mg |
| Biotin | 150 µg |
| Folsäure | 100 µg |
| Jod | 37,5 µg |
| Selen | 15 µg |

10. Zubereitung nach einem der vorstehenden Ansprüche, zur Verwendung als Arzneimittel, Nahrungsmittel, Nahrungsergänzungsmittel und Diätetikum.

11. Verwendung einer Zubereitung wie in einem der Ansprüche 1 bis 8 definiert zum Anregen und/oder Wiederbeleben des Wachstums von Oberhautgebilden, insbesondere der Haare und Nägel sowie zur Förderung der Zellneubildung der Haut.

12. Verwendung einer Zubereitung wie in einem der Ansprüche 1 bis 9 definiert zur Erhöhung der Haardichte, zur Stimulierung des Haarwuchses, zur Verhinderung des Haarverlusts oder zur Verhinderung der Bildung von Kopfschuppen.

13. Verwendung einer Zubereitung wie in einem der Ansprüche 1 bis 9 definiert zur Herstellung eines Arzneimittels zur Behandlung von krankhaft bedingtem Haarausfall, Alopecia areata, Alopecia diffusa, androgenetischer Alopezie, androgenetischem Haarausfall bei Frauen durch Behandlung hormonabhängiger Tumore, Alopecia praematura, Alopecia simplex oder Alopecia totalis.
